(19) Europäisches Patentamt European Patent Office Office européen des brevets

(11) **EP 3 453 309 B1**

(12) **EUROPÄISCHE PATENTSCHRIFT**

(45) Veröffentlichungstag und Bekanntmachung des Hinweises auf die Patenterteilung:
**18.03.2020 Patentblatt 2020/12**

(51) Int Cl.:
*A61B 1/00* *(2006.01)*     *A61B 17/00* *(2006.01)*
*H02N 2/04* *(2006.01)*

(21) Anmeldenummer: **18192169.3**

(22) Anmeldetag: **03.09.2018**

(54) **ENDOSKOPISCHES INSTRUMENT**

ENDOSCOPIC INSTRUMENT

INSTRUMENT ENDOSCOPIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.09.2017 DE 102017120793**

(43) Veröffentlichungstag der Anmeldung:
**13.03.2019 Patentblatt 2019/11**

(73) Patentinhaber: **Richard Wolf GmbH**
**75438 Knittlingen (DE)**

(72) Erfinder: **Teichtmann, B. Eng. Elmar**
**75015 Bretten (DE)**

(74) Vertreter: **Patentanwälte Vollmann Hemmer Lindfeld**
**Partnerschaft mbB**
**Wallstraße 33a**
**23560 Lübeck (DE)**

(56) Entgegenhaltungen:
**JP-B2- 5 253 852**     **KR-A- 20090 011 393**
**US-A- 5 372 124**     **US-A1- 2014 309 625**

**Beschreibung**

Die vorliegende Offenbarung betrifft ein endoskopisches Instrument zur minimalinvasiven medizinischen Behandlung oder Diagnose, das insbesondere geeignet ist, robotergesteuert geführt und betätigt zu werden.

Ein robotersteuerbares Endoskop ist beispielsweise aus der US 7,297,142 B2 bekannt. Dort wird ein Instrumentenkopf über eine Mehrzahl von Zugseilen abgeknickt und betätigt. Die Robotersteuerung über Zugseile ist allerdings technisch recht aufwändig.

Die US 2014/309625 A1 beschreibt endoskopisches Instrument, bei dem Piezo-Motoren an Seilzügen zur Steuerung eines Instrumentenkopfs verwendet werden.

In der US 5,372,124 A ist ein endoskopisches Biopsiezangeninstrument beschrieben, bei dem piezoelektrische Elemente zum Öffnen und Schließen von Biopsiezangenmaulteilen verwendet werden.

Die JP 5253852 B2 beschreibt ein endoskopisches Instrument mit einem mittels über eine Umlenkrolle geführter Seilzüge abwinkelbaren Instrumentenkopf, wobei die Umlenkrolle durch Ultraschallmotoren angetrieben wird.

Das hierin offenbarte endoskopische Instrument erlaubt dagegen eine genaue Robotersteuerung und -betätigung eines Instrumentenkopfs auf einfachere Weise.

Das hierin offenbarte endoskopische Instrument verzichtet nämlich auf Zugseile und weist einen länglichen Schaft und einem Instrumentenkopf am distalen Schaftende auf, der selbst bewegbar ist oder zumindest ein bewegbares Kopfteil aufweist. Das Instrument weist ferner mindestens zwei Paare oszillatorisch beweglicher Impulsgeber und mindestens zwei Übertragungsräder auf, wobei die Übertragungsräder koaxial zueinander und unabhängig voneinander drehbar auf einer quer zur Längsachse des Schafts verlaufenden Welle angeordnet sind, wobei ein erstes der Übertragungsräder der Umsetzung einer oszillatorischen Bewegung eines Impulsgebers eines ersten der Impulsgeberpaare in eine gleichgerichtete Rotations- oder Translationsbewegung in eine erste Bewegungsrichtung zur Bewegungssteuerung des Instrumentenkopfes oder des bewegbaren Kopfteils dient und der Umsetzung einer oszillatorischen Bewegung des anderen Impulsgebers des ersten Impulsgeberpaars in eine gleichgerichtete Rotationsoder Translationsbewegung in eine der ersten Bewegungsrichtung entgegengesetzte zweite Bewegungsrichtung zur Bewegungssteuerung des Instrumentenkopfes oder des bewegbaren Kopfteils dient. Außerdem dient ein zweites der Übertragungsräder der Umsetzung einer oszillatorischen Bewegung eines Impulsgebers eines zweiten der Impulsgeberpaare in eine gleichgerichtete Rotations- oder Translationsbewegung in eine zur ersten und zweiten Bewegungsrichtung komplementäre dritte Bewegungsrichtung zur Bewegungssteuerung des Instrumentenkopfes oder des bewegbaren Kopfteils sowie der Umsetzung einer oszillatorischen Bewegung des anderen Impulsgebers des zweiten Impulsgeberpaars in eine gleichgerichtete Rotations- oder Translationsbewegung in eine der dritten Bewegungsrichtung entgegengesetzte vierte Bewegungsrichtung zur Bewegungssteuerung des Instrumentenkopfes oder des bewegbaren Kopfteils. Des Weiteren ist mindestens das erste Übertragungsrad mit einem Kegelrad gekoppelt, das um die Längsachse des Schafts drehbar ist.

Der erste Impulsgeber kann beispielsweise ein oszillatorisch anregbarer piezoelektrischer Aktor am Instrumentenkopf sein oder ein Schub-/Zugmittel als Übertragungselement, das beispielweise proximalseitig von einem piezoelektrischen Aktor in eine oszillatorisch axiale Bewegung anregbar ist und diese oszillatorisch axiale Bewegung auf die Bewegungsumformereinheit überträgt. Zusätzlich oder alternativ könnte die oszillatorische Bewegung auch rotatorisch sein. Die Bewegungsumformereinheit kann dabei vorzugsweise wie ein Schrittmotor wirken, der schrittweise die gleichgerichtete Rotationsoder Translationsbewegung in eine erste Bewegungsrichtung zur Bewegungssteuerung des Instrumentenkopfes und/oder des bewegbaren Kopfteils bewirkt. Unter "oszillatorischer Bewegung" sei hier eine irgendwie ausgeartete einmalige oder mehrmalige Hin- und Herbewegung gemeint, d.h. es kann eine sinusförmige Schwingung sein oder gepulst, rechteckig, sägezahnförmig, kontinuierlich, diskontinuierlich, periodisch oder aperiodisch.

Die gleichgerichtete Rotations- oder Translationsbewegung kann beispielsweise ein Abknicken des Instrumentenkopfs um einen Polarwinkel $\theta$ bezüglich der Längsachse des Schafts sein, d.h. eine sogenannte Nickbewegung, oder eine Drehbewegung des Instrumentenkopfs um einen Azimutwinkel $\phi$ um die Längsachse des Schafts, d.h. eine sogenannte Rollbewegung, oder eine Öffnungs- bzw. Schließbewegung eines Zangen- oder Scherenmaulteils des Instrumentenkopfs um einen Öffnungswinkel $\delta$ sein, oder eine kontinuierliche Drehbewegung eines Bohrers, einer Fräse oder eines Schraubers im Instrumentenkopf.

Hier sollen die Begriffe "Bewegungsrichtung", "Richtung", "Impulsrichtung" bzw. "gerichtet" bei einer translatorischen Bewegung entlang eines Streckenvektors $\vec{s}$ als die Richtung des Geschwindigkeitsvektors $\vec{v} = \frac{d\vec{s}}{dt}$ verstanden werden und bei einer Drehbewegung um einen Winkel $\varphi$ um eine Achse durch das Vorzeichen der Drehgeschwindigkeit $\omega = \frac{d\varphi}{dt}$ definiert sein. "Gleichgerichtet" soll demnach bedeuten, dass sich die Richtung nicht mit der Oszillation der oszillatorischen Bewegung ändert. Die gleichgerichtete Rotations- oder Translationsbewegung kann allerdings auf Befehl vorzugsweise mittels weiterer Impulsgeber umgestellt, umgekehrt oder beliebig verändert werden.

Optional weist die Bewegungsumformereinheit ein erstes Übertragungsrad und ein erstes Kopplungselement auf, wobei das erste Kopplungselement während einer ersten Phase der oszillatorischen Bewegung des ersten Impulsgebers in eine erste Impulsrichtung mit dem Übertragungsrad kraftschlüssig gekoppelt ist und während einer zweiten Phase der oszillatorischen Bewegung des ersten Impulsgebers in eine der ersten Impulsrichtung entgegengesetzte zweite Impulsrichtung nicht mit dem ersten Übertragungsrad kraftschlüssig gekoppelt ist, sodass sich das erste Übertragungsrad bei oszillatorischer Bewegung des ersten Impulsgebers in eine gleichgerichtete erste Drehrichtung bewegt, welche die erste Bewegungsrichtung am distalen Schaftende zur Bewegungssteuerung des Instrumentenkopfes und/oder des bewegbaren Kopfteils definiert.

Das Übertragungsrad kann beispielsweise ein Zahnrad sein, in das eine Klinke als Kopplungselement radial ein- und ausgreifen kann. Zusätzlich oder alternativ kann das Übertragungsrad ein Reibrad sein, mit dem eine Reibbacke als Kopplungselement radial in kraftschlüssigen Reibschluss gebracht und aus dem kraftschlüssigen Reibschluss genommen werden kann.

Optional schiebt der erste Impulsgeber also in einer ersten Phase das erste Übertragungsrad über das erste eingekoppelte Kopplungselement um einen Winkel $\varphi$ weiter. In der zweiten Phase kann sich das ausgekoppelte Kopplungselement wieder zurückstellen, beispielsweise mittels Federkraft, oder wird vom ersten Impulsgeber zurückgezogen. Optional dreht sich das erste Übertragungsrad nicht in der zweiten Phase. Optional sorgt also pro Schwingungsperiode nur die erste Phase für einen Vorschub des Übertragungsrads. Im Falle eines Zahnrads mit einer Teilung t kann die Bewegungsamplitude a des ersten Impulsgebers im Bereich zwischen t und 2t liegen, also $t < a < 2t$ gelten, sodass pro Schwingungsperiode nur ein minimal inkrementeller Vorschub um einen Zahn erzielt wird. Das Zahnrad kann beispielsweise ein Stirnrad sein mit beliebig gearteter Verzahnung, wobei optional eine Sägeverzahnung verwendet werden kann, um eine Kraftkopplung in der zweiten Phase beim Zurückstellen des Kopplungselements zu vermeiden, wenn das Kopplungselement nicht vollständig aus der Verzahnung gehoben wird. Zusätzlich oder alternativ kann das Kopplungselement für das Zurückstellen in der zweiten Phase auch vollständig aus der Verzahnung gehoben werden, wobei beispielsweise eine Rechteckverzahnung verwendet werden kann.

Das Instrument weist in einer ersten Ausführungsform einen beweglichen zweiten Impulsgeber auf, wobei die Bewegungsumformereinheit dazu ausgestaltet ist, eine oszillatorische Bewegung des zweiten Impulsgebers in eine gleichgerichtete Rotations- oder Translationsbewegung in eine zweite Bewegungsrichtung zur Bewegungssteuerung des Instrumentenkopfes und/oder des bewegbaren Kopfteils umzusetzen. Die erste und die zweite Bewegungsrichtung sind dabei entgegengesetzt gerichtet. Wenn der erste Impulsgeber beispielsweise ein Abknicken des Instrumentenkopfs um einen Polarwinkel $\theta$ bezüglich der Längsachse des Schafts bewirkt, d.h. eine sogenannte Nickbewegung, dann kann beispielsweise der zweite Impulsgeber den Instrumentenkopf um einen Polarwinkel $-\theta$ zurückstellen. Analog könnte der erste Impulsgeber eine Drehbewegung des Instrumentenkopfs um einen Azimutwinkel $\phi$ um die Längsachse des Schafts, d.h. eine sogenannte Rollbewegung, bewirken und der zweite Impulsgeber die entgegengesetzte Rollbewegung um $-\phi$. Analog kann die erste Bewegungsrichtung eine Öffnungsbewegung eines Zangen- oder Scherenmaulteils des Instrumentenkopfs um einen Öffnungswinkel $\delta$ sein und die zweite Bewegungsrichtung eine Schließbewegung des Zangen- oder Scherenmaulteils des Instrumentenkopfs um einen Schließwinkel $-\delta$. Entsprechend kann mit dem zweiten Impulsgeber auch eine kontinuierliche Drehbewegung eines Bohrers, einer Fräse oder eines Schraubers im Instrumentenkopf in eine andere Drehrichtung als mit dem ersten Impulsgeber bewirkt werden. Vorteilhaft an dieser ersten Ausführungsform ist, dass für die Bewegungsumformereinheit sowie für den ersten und zweiten Impulsgeber im Wesentlichen identische bzw. zueinander symmetrische Bauteile verwendet werden können, was die Herstellungskosten senkt und den Herstellungsprozess vereinfacht. Die Bewegungsamplitude a des ersten Impulsgebers und die Bewegungsamplitude b des zweiten Impulsgebers können im Wesentlichen gleich sein.

Optional weist das Instrument in einer zweiten Ausführungsform einen beweglichen zweiten Impulsgeber auf, wobei die Bewegungsumformereinheit dazu ausgestaltet ist, mittels einer oszillatorischen Bewegung des zweiten Impulsgebers die Richtung der Rotations- oder Translationsbewegung zur Bewegungssteuerung des Instrumentenkopfes und/oder des bewegbaren Kopfteils zu vorzugeben, wobei eine Phasenverschiebung zwischen der oszillatorischen Bewegung des ersten Impulsgebers und des zweiten Impulsgebers die erste bzw. zweite Bewegungsrichtung zur Bewegungssteuerung des Instrumentenkopfes und/oder des bewegbaren Kopfteils definiert. In dieser zweiten Ausführungsform kann der erste Impulsgeber die für die Bewegungssteuerung des Instrumentenkopfes und/oder des bewegbaren Kopfteils in die erste sowie die zweite Bewegungsrichtung benötigte Impulsübertragung bewirken. Die Oszillation des zweiten Impulsgebers kann dazu genutzt werden, entweder das erste Kopplungselement oder das zweite Kopplungselement in der ersten (Schub-)Phase der Oszillation des ersten Impulsgebers mit dem Übertragungsrad in Kraftschluss zu bringen. Je nach Phasenverschiebung wird entweder das erste oder das zweite Kopplungselement in der ersten (Schub-)Phase eingekoppelt.

In beiden Ausführungsformen kann also optional ein zweites Kopplungselement vorgesehen sein, wobei das zweite Kopplungselement während einer ersten Phase der oszillatorischen Bewegung des zweiten Impulsgebers in eine erste Impulsrichtung mit dem Übertragungsrad kraftschlüssig gekoppelt ist und während einer zweiten Phase der oszillatorischen Bewegung des zweiten Impulsgebers in eine der ersten Impulsrichtung entgegengesetzten zweiten Impulsrichtung nicht mit dem ersten Übertragungsrad kraftschlüssig gekoppelt ist, sodass sich das erste Übertragungsrad bei oszillatorischer Bewegung des zweiten Impulsgebers in eine gleichgerichtete zweite Drehrichtung bewegt, welche die zweite Bewegungsrichtung zur Bewegungssteuerung des Instrumentenkopfes und/oder des bewegbaren Kopfteils definiert. In der ersten Ausführungsform kann bei gleichzeitiger Oszillation des ersten Impulsgebers und des zweiten Impulsgebers ein Feststellen bzw. eine Bremswirkung erzielt werden. In der zweiten Ausführungsform definiert bei gleichzeitiger Oszillation des ersten Impulsgebers und des zweiten Impulsgebers die Phasenverschiebung zwischen den Oszillationen die bewirkte Bewegungsrichtung. In der zweiten Ausführungsform kann sich jeweils immer eines der Kopplungselemente bei fehlender Oszillation des ersten und zweiten Impulsgebers in kraftschlüssigem Eingriff mit dem Übertragungsrad befinden und dieses feststellen. In der zweiten Ausführungsform ist daher eine Rechteckverzahnung bevorzugt, bei der ein Eingriff eines der Kopplungselemente bei fehlender Oszillation des ersten Impulsgebers bereits ein Feststellen bzw. eine Bremswirkung in beide Drehrichtungen des Übertragungsrads bewirken kann.

Optional sind das erste und das zweite Kopplungselement an jeweils diametral gegenüberliegenden Seiten des ersten Übertragungsrads mit diesem kraftschlüssig koppelbar. Dabei können der erste und zweite Impulsgeber in Längsrichtung des Schafts schwingen, und die Schwingung über das erste Kopplungselement oder zweite Kopplungselement im Wesentlichen tangential entweder oben oder unten am Übertragungsrad einkoppeln und damit die Drehrichtung des Übertragungsrads definieren.

Bei Verwendung einer Sägeverzahnung auf dem Übertragungsrad kann das Übertragungsrad zwei axiale Abschnitte aufweisen, wobei die Verzahnungsrichtung zwischen den beiden Abschnitten umgekehrt ist. Alternativ oder zusätzlich können auch zwei Übertragungsradteile mit umgekehrter Verzahnungsrichtung koaxial miteinander gekoppelt sein. Das erste Kopplungselement kann dann mit dem ersten Abschnitt bzw. Übertragungsradteil kraftschlüssig koppelbar sein und das zweite Kopplungselement dann entsprechend mit dem zweiten Abschnitt bzw. Übertragungsradteil.

Der erste Impulsgeber, der zweite Impulsgeber, das erste Übertragungsrad, das erste Kopplungselement und das zweite Kopplungselement bilden einen ersten Teilesatz, mit dem eine Rotations- oder Translationsbewegung in die erste Bewegungsrichtung und die zweite Bewegungsrichtung zur Bewegungssteuerung des Instrumentenkopfes und/oder des bewegbaren Kopfteils bewirkbar ist. Analog zum ersten Teilesatz weist das Instrument einen zweiten Teilesatz auf, mit dem eine Rotations- oder Translationsbewegung in eine zur ersten und zweiten Bewegungsrichtung komplementären dritten bzw. vierten Bewegungsrichtung zur Bewegungssteuerung des Instrumentenkopfes und/oder des bewegbaren Kopfteils bewirkbar ist. Wenn beispielsweise die erste und zweite Bewegungsrichtung eine Nickbewegung hin und her bewirkt, dann kann die komplementäre dritte bzw. vierte Bewegungsrichtung eine Rollbewegung hin und her sein.

Optional kann zum ersten und zweiten Teilesatz ein dritter Teilesatz vorgesehen sein, wobei mit dem dritten Teilesatz eine Rotations- oder Translationsbewegung in eine zur ersten, zweiten, dritten und vierten Bewegungsrichtung komplementären fünften bzw. sechsten Bewewegungsrichtung zur Bewegungssteuerung des Instrumentenkopfes und/oder des bewegbaren Kopfteils bewirkbar ist. Wenn beispielsweise die erste und zweite Bewegungsrichtung eine Nickbewegung hin und her bewirkt und die komplementäre dritte bzw. vierte Bewegungsrichtung eine Rollbewegung hin und her ist, dann kann die komplementäre fünfte bzw. sechste Bewegungsrichtung eine Öffnungs- bzw. Schließbewegung eines Zangen- oder Scherenmaulteils sein.

Die durch die unterschiedlichen Teilesätze bewirkbaren Bewegungen können vollkommen unabhängig voneinander und gleichzeitig stattfinden. Der zweite Teilesatz kann beispielsweise einen dritten Impulsgeber, einen vierten Impulsgeber, ein zweites Übertragungsrad, ein drittes Kopplungselement und ein viertes Kopplungselement aufweisen mit im Wesentlichen fast identischen Bauteilen. Der dritte Teilesatz kann beispielsweise einen fünften Impulsgeber, einen sechsten Impulsgeber, ein drittes Übertragungsrad, ein fünftes Kopplungselement und ein sechstes Kopplungselement aufweisen mit ebenfalls im Wesentlichen fast identischen Bauteilen. Dies senkt die Herstellungskosten und die Komplexität der Herstellung.

Lediglich die Kopplung der unterschiedlichen Übertragungsräder auf den Instrumentenkopf oder das bewegbare Kopfteil kann sich dabei unterscheiden. Die Übertragungsräder sind koaxial zueinander und unabhängig voneinander drehbar angeordnet auf einer gemeinsamen Welle. Die Welle verläuft quer zur Längsachse des Schafts, sodass eine axiale Schwingung der Impulsgeber in einfacher Weise tangential auf die Übertragungsräder eingekoppelt werden kann. Für die Nickbewegung kann beispielsweise ein außen liegendes zweites Übertragungsrad fest mit dem Instrumentenkopf verbunden sein, um den Instrumentenkopf um die Welle zu verschwenken und damit um einen Polarwinkel θ gegenüber der Längsachse des Schafts abzuknicken.

Für die Rollbewegung kann das erste Übertragungsrad mit einem ersten Kegelrad gekoppelt sein, das um die Längsachse des Schafts drehbar ist. Das erste Kegelrad kann fest mit dem Instrumentenkopf verbunden sein, um den Instrumentenkopf um einen Azimutwinkel φ um die Längsachse des Schafts zu drehen. Für die Öffnungs- und Schließbewegung der Zangenmaulteile kann das dritte Übertragungsrad mit einem zweiten Kegelrad gekoppelt sein, das ebenfalls um die Längsachse des Schafts drehbar ist und vorzugsweise eine durch das erste Kegelrad durchgreifendes Betätigungselement antreibt. Das Betätigungselement kann beispielsweise axial verschoben werden zum Aufhebeln bzw. Einschwenken eines Zangen- oder Scherenmaulteils. Alternativ oder zusätzlich kann das Zangen- oder Scherenmaulteil über eine Schraubverzahnung mit dem Betätigungselement gekoppelt sein und sich damit bei Drehung des Betätigungselements in einer Richtung öffnen und bei Drehung des Betätigungselements in die andere Richtung schließen.

Optional weist das Instrument mindestens einen piezoelektrischen Aktor zum oszillatorischen Antrieb mindestens eines Impulsgebers auf. Optional ist der Impulsgeber selbst ein piezoelektrischer Aktor im distalen Bereich des Instruments, sodass nur elektrische Signale und keine mechanischen Signale über die Länge des Schafts übertragen werden müssen. Alternativ dazu kann der piezoelektrische Aktor in einem proximalen Bereich des Instruments angeordnet sein, beispielsweise wenn der Bauraum am distalen Ende nicht ausreicht. In diesem Fall können die Impulsgeber sich im Wesentlichen über die Länge des Schafts erstreckende Schubmittel zur mechanischen Übertragung einer axialen Schwingung vom proximalen Schaftende zum distalen Schaftende sein. An seinem proximalen Ende kann dann jeder Impulsgeber durch einen eigenen piezoelektrischen Aktor zu axialen Schwingungen angeregt werden, um diese dann an seinem distalen Ende auf ein jeweiliges Kopplungselement zu übertragen.

Wie bereits zuvor erwähnt, kann die gleichgerichtete Rotations- oder Translationsbewegung in eine erste Bewegungsrichtung eine Abwinklung (Nickbewegung) des Instrumentenkopfes oder eine Arbeitsbewegung eines bewegbaren Kopfteils bewirken. Alternativ oder zusätzlich kann die gleichgerichtete Rotations- oder Translationsbewegung in eine erste Bewegungsrichtung eine Rotation (Rollbewegung) des Instrumentenkopfes um eine Längsachse des Schafts oder eine kontinuierliche Arbeitsdrehbewegung eines bewegbaren Kopfteils bewirken. Optional kann die gleichgerichtete Rotations- oder Translationsbewegung in eine erste Bewegungsrichtung eine Öffnungsbewegung und in eine zweite Bewegungsrichtung eine Schließbewegung eines Zangen- oder Scherenmaulteils des Instrumentenkopfs bewirken.

Nachfolgend wird das hierin offenbarte endoskopische Instrument beispielhaft anhand der beigefügten Figuren beschrieben. In diesen zeigt:

Fig. 1 eine perspektivische Ansicht auf ein robotergesteuertes System mit drei endoskopischen Instrumenten gemäß einer beispielhaften Ausführungsform der vorliegenden Offenbarung;

Fig. 2 eine perspektivische Ansicht auf einen distalen Endbereich eines endoskopischen Instruments gemäß einer beispielhaften Ausführungsform der vorliegenden Offenbarung;

Fig. 3 eine Unteransicht auf einen distalen Endbereich eines endoskopischen Instruments gemäß einer beispielhaften Ausführungsform der vorliegenden Offenbarung;

Fig. 4 eine perspektivische Ansicht auf einen distalen Endbereich eines ersten Teilesatzes eines endoskopischen Instruments gemäß einer ersten beispielhaften Ausführungsform der vorliegenden Offenbarung;

Fig. 5 eine perspektivische Detailansicht auf die Kopplung zwischen einem Übertragungsrad und einem Kopplungselement eines endoskopischen Instruments gemäß einer ersten beispielhaften Ausführungsform der vorliegenden Offenbarung;

Fig. 6 eine Seitenansicht auf einen ersten Teilesatz (ohne Impulsgeber) eines endoskopischen Instruments gemäß einer zweiten beispielhaften Ausführungsform der vorliegenden Offenbarung;

Fig. 7 eine perspektivische Ansicht von einer ersten Seite auf einen ersten Teilesatz (ohne Impulsgeber) eines endoskopischen Instruments gemäß einer zweiten beispielhaften Ausführungsform der vorliegenden Offenbarung;

Fig. 8 eine perspektivische Ansicht von einer zweiten Seite auf einen ersten Teilesatz (ohne Impulsgeber) eines endoskopischen Instruments gemäß einer zweiten beispielhaften Ausführungsform der vorliegenden Offenbarung;

Fig. 9 eine Seitenansicht auf ein Schieberelement eines endoskopischen Instruments gemäß einer zweiten beispielhaften Ausführungsform der vorliegenden Offenbarung;

Fig. 10 eine Seitenansicht auf ein Umstellelement eines endoskopischen Instruments gemäß einer zweiten beispielhaften Ausführungsform der vorliegenden Offenbarung;

Fig. 11 und 12 schematische Diagramme der Auslenkung der Kopplungselemente über die Zeit gemäß der zweiten beispielhaften Ausführungsform der vorliegenden Offenbarung;

Fig. 13 eine perspektivische Detailansicht auf geöffnete Zangenmaulteile am Kopf eines endoskopischen Instruments gemäß einer beispielhaften Ausführungsform der vorliegenden Offenbarung; und

Fig. 14 eine Detailquerschnitt auf geschlossene Zangenmaulteile am Kopf eines endoskopischen Instruments gemäß einer beispielhaften Ausführungsform der vorliegenden Offenbarung.

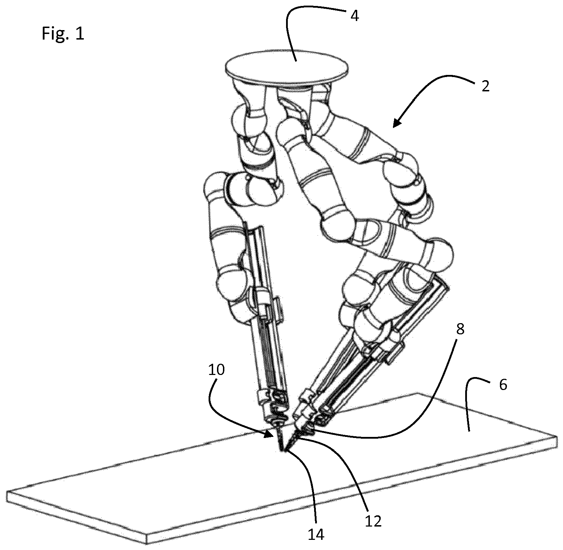

In Fig. 1 ist ein robotergesteuertes medizinisches Operationssystem dargestellt mit drei jeweils als Mehrgelenkarm ausgebildeten Roboterarmen 2. Die Roboterarme 2 sind jeweils an einer Platte 4, die vertikal oberhalb eines Operationstisches 6 angeordnet ist, befestigt. An ihrem distalen Ende weisen die Roboterarme 2 jeweils eine Schnittstelle 8 auf, an der ein endoskopisches Instrument 10 mit dem Roboterarm 2 verbunden ist. Bei dem Instrument 10 handelt es sich um ein Schaftinstrument mit einem länglichen starren Schaft 12, an dessen distalen Ende ein Instrumentenkopf 14 angeordnet ist.

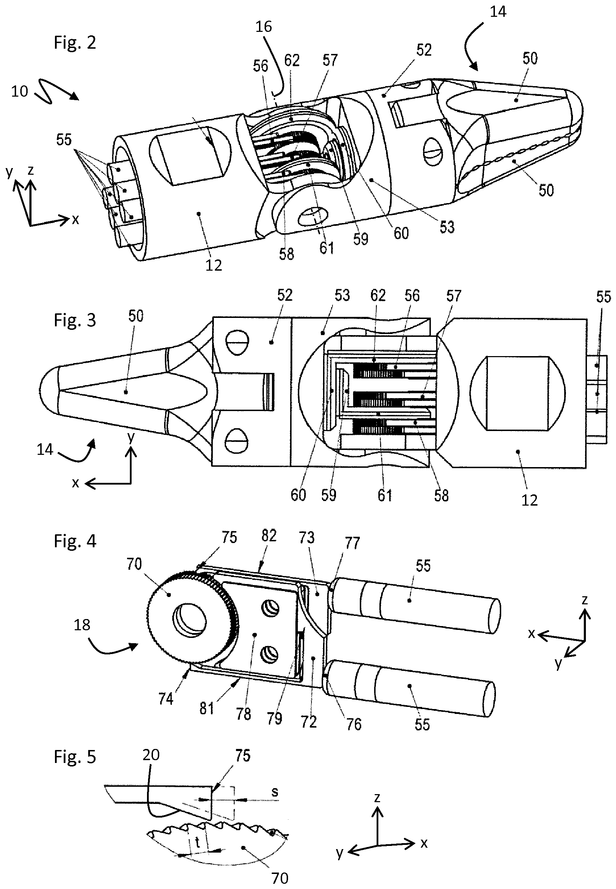

Fig. 2 zeigt einen distalen Endbereich des endoskopischen Instruments 10 detaillierter. Das Instrument 10 kann mittels des Roboterarms 2, mit dem es verbunden ist, in jede beliebige Raumrichtung orientiert werden. Zum besseren Verständnis der Funktionsweise des Instruments 10 ist ein lokales rechtshändiges kartesisches Koordinatensystem angegeben, das mit dem endoskopischen Instrument 10 fest verbunden ist. Die x-Achse entspricht dabei der Längsachse des Schafts 12 zum distalen Ende hin gerichtet. Eine Drehung um die x-Achse bzw. Längsachse des Schafts 12 wird hierin eine Rollbewegung genannt und die x-Achse selbst ggf. die Roll-Achse. Die laterale y-Achse steht senkrecht zur x-Achse, wobei eine Drehung um die y-Achse eine Nickbewegung genannt wird bzw. die y-Achse selbst die Nick-Achse. Die zur x-Achse und y-Achse senkrecht stehende z-Achse wird auch Gier-Achse genannt, wobei eine Drehung um die Gier-Achse eine Gierbewegung ist.

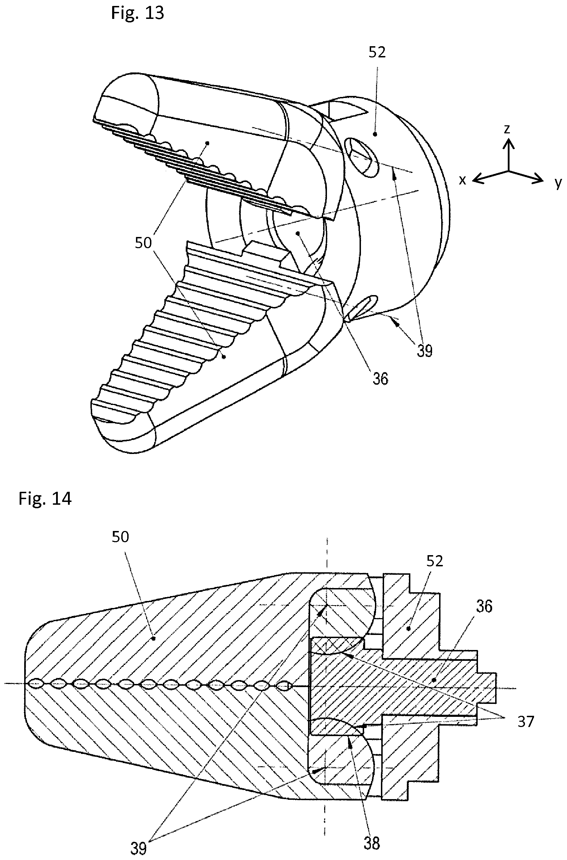

Ein Verbindungsteil 53 ist am distalen Ende des Schaftes 12 über eine sich in y-Richtung erstreckende Welle 16 angelenkt, wobei das Verbindungsteil 53 gegenüber dem Schaft 12 eine Nickbewegung in beide Drehrichtungen um die Nick-Achse y vollführen kann. Eine Maulteilbasis 52 ist mit dem distalen Ende des Verbindungsteils 53 drehbar um die Roll-Achse x verbunden. Ein Maulteilpaar 50 bildet hier den Instrumentenkopf 14 in Form einer Zange. Die Maulteile 50 sind distalseitig an der Maulteilbasis 52 angelenkt und können über eine Schwenkbewegung um jeweils zur Nickachse y parallele Schwenkachsen 39 (siehe Figuren 13 und 14) in eine Öffnungs- und Schließposition gebracht werden. Die Maulteile 50 sind Kopfteile, die hier als Zangenmaulteile gezeigt sind, allerdings auch Scherenmaulteile, Bohrer, Schrauber, Fräsen oder bewegliche Kopfteile anderer Art sein können.

Innerhalb des Schafts 12 sind parallel zueinander sechs längliche Impulsgeber 55 angeordnet, die jeweils über eine axiale Bewegung in x-Richtung einen Bewegungsimpuls übertragen. Proximalseitig können die Impulsgeber 55 jeweils beispielsweise mit einem Piezo-Element in axiale Schwingung bzw. Oszillation versetzt werden, um Bewegungsimpulse zu übertragen. Die Impulsgeber 55 können allerdings auch selbst am distalen Ende des Schaftes 12 angeordnete Piezo-Elemente sein. Hier sind die Impulsgeber 55 Übertragungselemente, die Schwingungen von proximalseitig vom Schaft angeordneten Piezo-Elementen übertragen. Die sechs länglichen Impulsgeber 55 bilden drei Impulsgeberpaare, wobei ein erstes der Impulsgeberpaare eine Rollbewegung der Maulteilbasis 52 im Uhrzeigersinn bzw. entgegen dem Uhrzeigersinn bewirkt. Ein zweites der Impulsgeberpaare bewirkt eine Öffnungs- bzw. Schließbewegung der Maulteile 50, und ein drittes der Impulsgeberpaare bewirkt eine Nickbewegung des Verbindungsteils 53 gegenüber dem Schaft 12. Eine Gier-Bewegung kann durch eine Kombination aus einer Rollbewegung um 90° und einer Nickbewegung erzeugt werden, sodass es dafür keines eigenen Impulsgeberpaares bedarf. Ein Impulsgeber 55 überträgt dabei nur in einer Schwingungsrichtung (hier distalwärts durch Schieben) und nicht in beiden Schwingungsrichtungen (hier also nicht proximalwärts durch Ziehen) Bewegungsimpulse. Daher wird hier ein Impulsgeberpaar eingesetzt, bei dem eine Bewegungsrichtung durch einen Impulsgeber 55 eines Paares bewirkt wird und die entgegengesetzte Bewegungsrichtung durch den anderen Impulsgeber 55 des Paares.

Im Folgenden wird erläutert wie eine oszillatorische Bewegung eines Impulsgebers in eine gleichgerichtete Rotations- oder Translationsbewegung zur Bewegungssteuerung des Instrumentenkopfes 14 und/oder des bewegbaren Kopfteils 50 umgesetzt wird. Dies geschieht mittels einer Bewegungsumformereinheit 18. Die Bewegungsumformereinheit 18 weist dazu drei Übertragungsräder 70 (siehe Figuren 2 und 3) und sechs Kopplungselemente 74, 75 in Form von drei Kopplungselementpaaren 56, 57, 58. Jedes Kopplungselementpaar 56, 57, 58 ist einem der Impulsgeberpaare zugeordnet und kann von diesem in axiale Schwingungen versetzt werden.

Jedes der Übertragungsräder 70 ist wiederum einem der Kopplungselementpaare 56, 57, 58 zugeordnet, um eine oszillatorische Bewegung eines der ihm zugeordneten Impulsgeber 55 in eine gleichgerichtete Rotations- oder Translationsbewegung zur Bewegungssteuerung des Instrumentenkopfes 14 und/oder des bewegbaren Kopfteils 50 umzusetzen. Dabei ist ein Kopplungselement 74 während einer ersten Phase der oszillatorischen Bewegung (hier distalwärts schiebend) mit dem Übertragungsrad 70 kraftschlüssig gekoppelt und während einer zweiten Phase oszillatorischen Bewegung (hier proximalwärts ziehend) nicht mit dem Übertragungsrad 70 kraftschlüssig gekoppelt, sodass sich das Übertragungsrad 70 bei oszillatorischer Bewegung des ersten Impulsgebers 55 schubweise in eine gleichgerichtete Drehrichtung bewegt. Diese gleichgerichtete Drehrichtung definiert die Bewegungsrichtung am distalen Schaftende zur Bewegungssteuerung des Instrumentenkopfes 14 und/oder des bewegbaren Kopfteils 50. Um die entgegengesetzte Bewegungsrichtung am distalen Schaftende zur Bewegungssteuerung des Instrumentenkopfes 14 und/oder des bewegbaren Kopfteils 50 zu bewirken, wird das gleiche Übertragungsrad 70 mittels des anderen Kopplungselements 75 des Kopplungselementpaares schubweise in die entgegengesetzte Drehrichtung bewegt. Ein separater Impulsgeber 55 erzeugt dabei die oszillatorische Bewegung des anderen Kopplungselements 75 des Kopplungselementpaares 56, 57, 58.

In Figur 3 sind drei im Wesentlichen identische Übertragungsräder 70 koaxial und äquidistant auf der Welle 16 angeordnet. Jedes Übertragungsrad 70 weist dabei einen ersten und einen zweiten Axialabschnitt auf, wobei der Mantel des ersten Axialabschnitts ein im Uhrzeigersinn ausgerichtetes Sägezahnprofil aufweist und der Mantel des zweiten Axialabschnitts ein entgegen dem Uhrzeigersinn ausgerichtetes Sägezahnprofil. Der erste und zweite Axialabschnitt sind im Wesentlichen gleich groß und haben, bis auf die Ausrichtung, im Wesentlichen das gleiche Sägezahnprofil.

Das in Figur 3 unten liegende Übertragungsrad 70 ist drehfest mit dem Verbindungsteil 53 verbunden, sodass eine Drehbewegung dieses Übertragungsrads 70 eine Nickbewegung des Instrumentenkopfes 14 bewirkt. Das obere Übertragungsrad 70 ist drehfest mit einem Kegelzahnrad 62 verbunden, welches mit einem äußeren Kegelzahnrad 60 verzahnt ist, dessen Drehachse entlang der Rollachse x liegt und drehfest mit der Maulteilbasis 52 verbunden ist. Eine Drehbewegung des in Figur 3 oben liegenden Übertragungsrads 70 bewirkt damit eine Rollbewegung des Instrumentenkopfes 14. Anders als die Nickbewegung, die nur bis zu einem maximalen Auslenkungswinkel $\theta$ durchgeführt werden kann, ist die Rollbewegung unendlich in eine Richtung durchführbar. Damit kann beispielsweise auch ein Bohrer oder Schrauber als Werkzeug eines Instrumentenkopfes 14 betätigt werden. Das mittlere Übertragungsrad 70 ist drehfest mit einem Kegelzahnrad 61 verbunden, welches mit einem inneren Kegelzahnrad 59 verzahnt ist, dessen Drehachse entlang der Rollachse x liegt und mit einem durch die Maulteilbasis 52 geführten Betätigungselement 36 (siehe Fig. 14) gekoppelt ist. Das Betätigungselement 36 kann beispielsweise drehfest mit dem inneren Kegelzahnrad 59 verbunden sein und am distalen Ende radial eine Schrägverzahnung 38 aufweisen, die in entsprechende Schrägverzahnungen 37 an den Maulteilen 50 greifen, um bei einer Drehung des inneren Kegelzahnrads 59 bzw. Betätigungselements 36 um die x-Achse eine Öffnungs- bzw. Schließbewegung durch Verschwenken der Maulteile 50 um zur y-Achse parallele Schwenkachsen 39 (siehe Figuren 13 und 14) zu bewirken.

Figur 4 zeigt detaillierter die Wirkungsweise der Bewegungsumformereinheit 18 für ein Übertragungsrad 70 und ein zugehöriges Kopplungselementpaar 74, 75, das von zwei Impulsgebern 55 angesteuert wird. Die anderen beiden Übertragungsräder 70 samt zugehörigen Kopplungselementpaaren sind im Wesentlichen identisch dazu ausgestaltet und unabhängig voneinander drehbar. Jedes Übertragungsrad 70 bildet zusammen mit dem zugehörigen Kopplungselementpaar 74, 75 und den zwei Impulsgebern 55 einen von drei im Wesentlichen baugleichen Teilesätzen, von denen jeweils ein erster Teilesatz die Rollbewegung rechts- oder links herum bewirkt, ein zweiter Teilesatz die Nickbewegung von der Längsachse weg und zur Längsachse hin bewirkt und ein dritter Teilesatz die Öffnungs- und Schließbewegung der Maulteile 50 bewirkt.

Die Kopplungselemente 74, 75 sind im Wesentlichen jeweils Klinken oder Stirnflächen eines L-förmigen Federbügels 81, 82 der über ein federndes Festkörpergelenk 79 an jeweils einem im Wesentlichen flächigen Grundkörper 72, 73 angelenkt ist. Die im Wesentlichen flächigen Grundkörper 72, 73 können in Form von Blechen ausgestaltet sein, wobei die flächigen Grundkörper 72, 73 parallel zueinander in der xz-Ebene verlaufen. Wird der L-förmige Federbügel 81 (in Figur 4 vorne liegend) vom unteren Impulsgeber 55 in x-Richtung angestoßen, so schwenkt der L-förmige Federbügel 81 um das Festkörpergelenk 79. Das sich am distalen Ende des Federbügels 81 befindende untere Kopplungselement 74 greift bei Verschwenkung des Federbügels 81 um das Festkörpergelenk 79 in das Sägezahnprofil des vorderen Axialabschnitts des Übertragungsrads 70 (siehe Fig. 5) und überträgt einen Impuls in x-Richtung auf das Übertragungsrad 70. Das Sägezahnprofil des vorderen Axialabschnitts des Übertragungsrads 70 weist dazu Steilflächen mit einer Normalen auf, die am Eingriffspunkt des Kopplungselements 74 in negative x-Richtung zeigen, sodass das Kopplungselement 74 kraftschlüssig in das Sägezahnprofil eingreift und das Übertragungsrad 70 anschiebt zu einer Drehung im Uhrzeigersinn. Diese Drehung erfolgt schubweise, wobei der axiale Bewegungspfad s des Kopplungselements 74 in x-Richtung mindestens einer Teilung t des Sägezahnprofils entspricht, um das Übertragungsrad 70 um mindestens einen Zahn weiterzuschieben. Der Federbügel 81 weist am distalen Ende zur negativen x-Richtung hin eine Schrägfläche 20 auf, die mit den Schrägflächen des Sägezahnprofils in Gleitkontakt steht. Dadurch besteht beim federnden Zurückschwingen des Federbügels 81 kein Kraftschluss zwischen dem Federbügel 81 und dem Übertragungsrad 70, sodass dieses nicht entgegen dem Uhrzeigersinn zurückgedreht wird. Der untere Impulsgeber 55 überträgt einen axialen Schwingungsimpuls in x-Richtung mittels einer Kontaktfläche 76 auf den Federbügel 81, um diesen zu verschwenken.

Dazu im Wesentlichen rotationssymmetrisch bezüglich der x-Achse ist das in Figur 4 obere Kopplungselement 75 am distalen Ende eines L-förmigen Federbügels 82 angeordnet, der über ein federndes Festkörpergelenk (nicht sichtbar) am flächigen Grundkörper 73 angelenkt ist. Der Federbügel 82 greift mit dem distalseitigen Kopplungselement 75 über eine Verschwenkung um das Festkörpergelenk in den hinteren Axialabschnitt des Übertragungsrads 70 mit gegenläufigem Sägezahnprofil, um eine Drehbewegung entgegen dem Uhrzeigersinn zu bewirken. Der obere Impulsgeber 55 überträgt einen axialen Schwingungsimpuls in x-Richtung mittels einer Kontaktfläche 77 auf den Federbügel 82, um diesen zu verschwenken. Sowohl das untere Kopplungselement 74 als auch das obere Kopplungselement 75 sind bei fehlender Anregung durch die jeweils zugeordneten Impulsgeber 55 nicht in kraftschlüssigem Eingriff mit dem Übertragungsrad 70, sondern werden erst mit der Verschwenkung des Federbügels 81 bzw. 82 in kraftschlüssigen Eingriff gebracht. Die jeweiligen federnden Festkörpergelenke stellen die Federbügel 81, 82 beim Zurückschwingen der Impulsgeber 55 in negative x-Richtung oder bei fehlender Anregung wieder in eine nicht eingreifende Ausgangsposition zurück. Um das Übertragungsrad 70 in eine oder beide Richtungen festzustellen, kann über eine dauerhaft angelegte Spannung am jeweils zugehörigen Piezoelement einer bzw. beide der Impulsgeber 55 in einer Auslenkungsstellung in x-Richtung und somit eine oder beide Stirnflächen 74, 75 in Eingriff mit dem Übertragungsrad 70 gehalten werden. Damit bewirkt ein gleichzeitiger Vorschub beider Impulsgeber 55 eine Bremswirkung auf das Übertragungsrad 70. Außerdem lässt sich die axiale Kraft, die benötigt wird, um das Übertragungsrad 70 zu bewegen, über das Piezoelement messen und ggf. anzeigen oder zur Werkzeugsteuerung verwenden. Beispielsweise können zur Vermeidung von unbeabsichtigter Zerstörung von Körpergewebe maximale Kräfte oder Drehmomente eingestellt werden, die nicht überschritten werden dürfen. Die beiden Axialabschnitte des Übertragungsrads 70 können auch durch zwei drehfest miteinander koaxial verbundene Übertragungsradteile realisiert sein. Der Kraftschluss zwischen den Federbügeln 81, 82 und dem Übertragungsgrad 70 kann auch durch Reibschluss erfolgen, sodass es dann keiner Sägeverzahnung bedarf und daher auch keiner zwei Axialabschnitte mit jeweils gegenläufiger Sägeverzahnung.

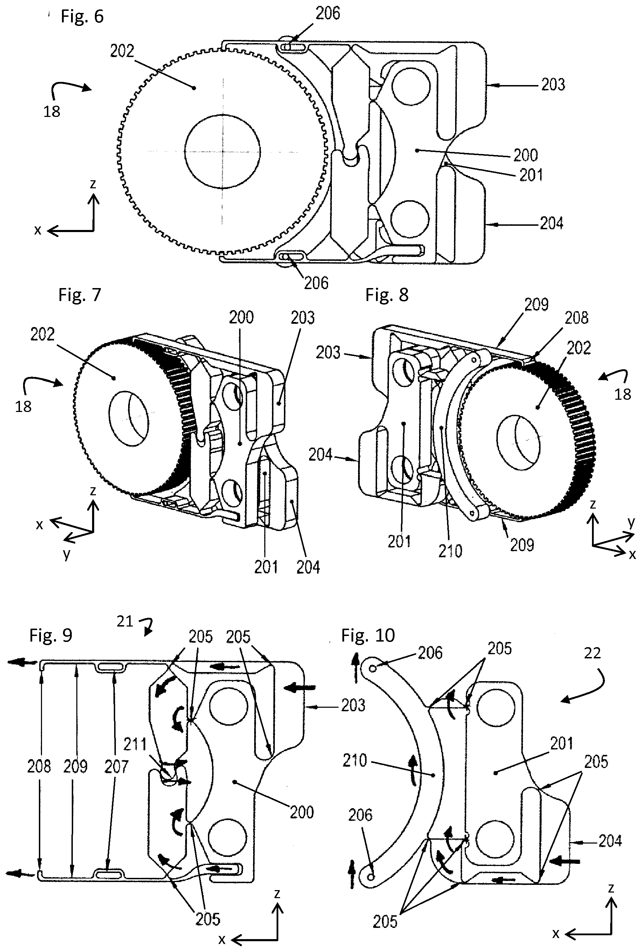

In Figuren 6 bis 10 ist eine zweite Ausführungsform der Bewegungsumformereinheit 18 gezeigt, bei der es ebenfalls keiner zwei Axialabschnitte des Übertragungsrads mit jeweils gegenläufiger Sägeverzahnung bedarf. Das Übertragungsrad 202 hat hier eine Rechteckverzahnung. Anders als im vorhergehenden Ausführungsbeispiel sind die Kopplungselemente 208 hier jeweils mit beiden Impulsgebern 55 über ein Schieberelement 21 und ein Umstellelement 22 gekoppelt (siehe Figuren 9 und 10). Ein in Figur 6 vorne liegender flächiger Grundkörper 200 des Schieberelements 21 wird hier mittels eines ersten oberen Impulsgebers 55 (nicht in Figur 6 gezeigt, analog zu Figur 4) über eine obere Kontaktfläche 203 angeregt. Ein in Figur 6 hinten liegender flächiger Grundkörper 201 des Umstellelements 22 (siehe Figur 10) wird mittels eines zweiten unteren Impulsgebers 55 (nicht in Figur 6 gezeigt, analog zu Figur 4) über eine untere Kontaktfläche 204 angeregt. Wie in Figur 9 gezeigt, sind die Kopplungselemente 208 hier derart mit der oberen Kontaktfläche 203 gekoppelt, dass ein Impuls in x-Richtung sowohl das obere als auch das untere Kopplungselement 208 in x-Richtung verschiebt. Die Kopplungselemente 208 sind hier radial in die Rechteckverzahnung des Übertragungsrads 202 passende Eingriffsabschnitte, die am distalen Ende von sich im Wesentlichen in x-Richtung erstreckenden Federbügeln 209 radial zum Übertragungsrad 202 hin ragen. Über eine Mehrzahl von Festkörpergelenken 205 wird eine Bewegung der Kontaktfläche 203 in x-Richtung auf eine gleichzeitige Bewegung der beiden Kopplungselemente 208 in x-Richtung übertragen (siehe Figur 9).

Die Federbügel 209 des Schieberelements 21 weisen hier Führungsnuten 207 auf, in die jeweils Führungsstifte 206 des Umstellelements 22 greifen, die über einen in Figur 6 hinten liegenden flächigen Grundkörper 201 mit der unteren Kontaktfläche 204 gekoppelt sind (siehe Figur 10). Über eine Mehrzahl von Festkörpergelenken 205 wird eine Bewegung der Kontaktfläche 204 in x-Richtung auf eine gleichzeitige Bewegung der beiden Führungsstifte 206 in z-Richtung übertragen (siehe Figur 10). Das bedeutet, dass eine Betätigung des unteren Impulsgebers 55 auf die Kontaktfläche 204 in x-Richtung das untere der Kopplungselemente 208 nach oben in Eingriff mit der Rechteckverzahnung des Übertragungsrads 202 bringt und das obere der Kopplungselemente 208 nach oben aus einem Eingriff mit der Rechteckverzahnung des Übertragungsrads 202 heraushebt. In einer Neutralstellung ohne Betätigung der Impulsgeber 55 ist nur das obere der Kopplungselemente 208 in Eingriff mit der Rechteckverzahnung des Übertragungsrads 202 (siehe Figur 6) und wirkt somit als Feststellbremse. Eine schubweise Drehung des Übertragungsrads 202 ist nur mit einer koordinierten Schwingung beider Impulsgeber 55 möglich, wobei die Phasenverschiebung zwischen der Schwingung des oberen Impulsgebers 55 und der Schwingung des unteren Impulsgebers 55 die Drehrichtung bestimmt. Wird beispielsweise ausgehend von einer Neutralstellung, bei der sich das obere der Kopplungselemente 208 in Eingriff mit der Rechteckverzahnung des Übertragungsrads 202 befindet, zunächst mit dem oberen Impulsgeber 55 die obere Kontaktfläche 203 betätigt, so wird das Übertragungsrad 202 in der Figur 6 entgegen dem Uhrzeigersinn gedreht. Sobald oder kurz bevor der Federbügel 209 in negative x-Richtung zurückfedert, wird mit dem unteren Impulsgeber 55 die untere Kontaktfläche 204 betätigt, sodass das obere der Kopplungselemente 208 aus dem kraftschlüssigen Eingriff gehoben wird und der Federbügel 209 in negative x-Richtung zurückfedern kann, ohne dabei das Übertragungsrad 202 wieder im Uhrzeigersinn zurückzudrehen. Danach kann die untere Kontaktfläche 204 in die Neutralstellung zurückfedern, um für den nächsten Vorschub das obere der Kopplungselemente 208 wieder in Eingriff mit der Rechteckverzahnung des Übertragungsrads 202 zu bringen.

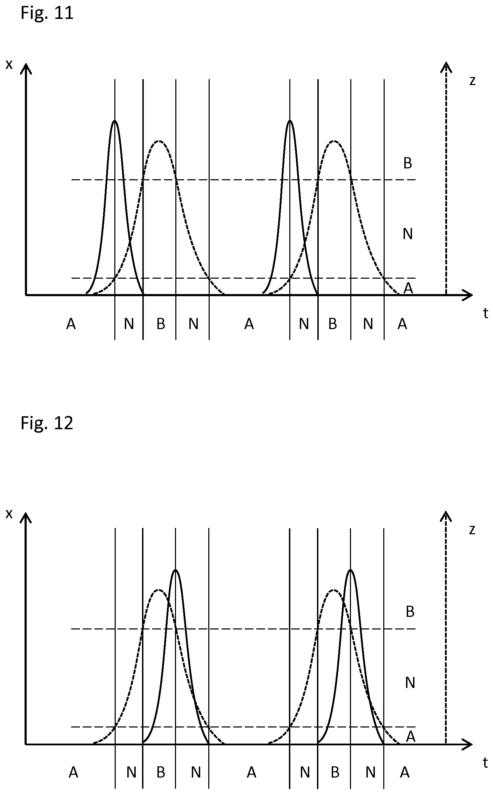

Im Folgenden wird anhand der Figuren 11 und 12 erläutert wie in diesem Ausführungsbeispiel eine Phasenverschiebung zwischen der oszillatorischen Bewegung des ersten oberen Impulsgebers 55 und des zweiten unteren Impulsgebers 55 die Drehrichtung des Übertragungsrads 202 und damit die Bewegungsrichtung zur Bewegungssteuerung des Instrumentenkopfes 14 und/oder des bewegbaren Kopfteils 50 definiert. In Figuren 11 und 12 ist schematisch eine Auslenkung der Kopplungselemente 208 in x-Richtung über die Zeit t (durchgezogene Linie) und eine Auslenkung der Kopplungselemente 208 in z-Richtung über die Zeit t abgetragen (gestrichelte Linie). Mit A sind Auslenkungen und entsprechende Zeiten bezeichnet, bei denen sich das obere der Kopplungselemente 208 in kraftschlüssigem Eingriff mit der Rechteckverzahnung des Übertragungsrads 202 befindet. Mit B sind Auslenkungen und entsprechende Zeiten bezeichnet, bei denen sich das untere der Kopplungselemente 208 in kraftschlüssigem Eingriff mit der Rechteckverzahnung des Übertragungsrads 202 befindet. Mit N sind Auslenkungen und entsprechende Zeiten bezeichnet, bei denen sich keines der Kopplungselemente 208 in kraftschlüssigem Eingriff mit der Rechteckverzahnung des Übertragungsrads 202 befindet. In Figur 11 ist die Phasenverschiebung zwischen den Impulsgebern 55 so gewählt, dass während des kraftschlüssigen Eingriffs des oberen der Kopplungselemente 208 (Abschnitte A) das Übertragungsrad 202 entgegen dem Uhrzeigersinn (in Figur6) geschoben wird. Das obere der Kopplungselemente 208 schwingt durch Federkraft zurück während sich keines der Kopplungselemente 208 in kraftschlüssigem Eingriff mit der Rechteckverzahnung des Übertragungsrads 202 befindet (Abschnitte N). In Figur 12 hingegen ist die Phasenverschiebung zwischen den Impulsgebern 55 so gewählt, dass während des kraftschlüssigen Eingriffs des unteren der Kopplungselemente 208 (Abschnitte B) das Übertragungsrad 202 im Uhrzeigersinn (in Figur6) geschoben wird. Das untere der Kopplungselemente 208 schwingt hier ebenfalls durch Federkraft zurück während sich keines der Kopplungselemente 208 in kraftschlüssigem Eingriff mit der Rechteckverzahnung des Übertragungsrads 202 befindet (Abschnitte N).

Auch in dem Ausführungsbeispiel der Figuren 6 bis 12 lässt sich die axiale Kraft, die benötigt wird, um das Übertragungsrad 70 zu bewegen, über ein Piezoelement messen und ggf. anzeigen oder zur Werkzeugsteuerung verwenden. Beispielsweise können zur Vermeidung von unbeabsichtigter Zerstörung von Körpergewebe maximale Kräfte oder Drehmomente eingestellt werden, die nicht überschritten werden dürfen. Der Kraftschluss zwischen den Federbügeln 81, 82 und dem Übertragungsgrad 70 kann auch hier durch Reibschluss erfolgen, sodass es dann keiner Rechteckverzahnung bedarf. Die Verzahnung muss hier keine Rechteckverzahnung sein, sondern kann auch eine Verzahnung mit beispielsweise schrägen und/oder abgerundeten Zahnflanken und schmalem Zahnkopf sein (ggf. eine Dreieckverzahnung oder Evolventenverzahnung), um ein unbeabsichtigtes "Aufsitzen" der Kopplungselemente auf dem Zahnkopf zu vermeiden.

In Figuren 13 und 14 wird das Öffnen und Schließen der Zangenmaulteile 50 deutlich. Die Zangenmaulteile 50 sind jeweils über parallel zur y-Achse verlaufende Schwenkachsen 39 an der Maulteilbasis 52 angelenkt. Das innere Kegelrad 59 (siehe Figur 4) ist kraftschlüssig mit dem Betätigungselement 36 gekoppelt, um eine Öffnungs- oder Schließbewegung der Zangenmaulteile 50 zu bewirken. Das innere Kegelrad 59 kann dabei drehfest mit dem Betätigungselement 36 verbunden sein, sodass sich das Betätigungselement 36 mit dem innere Kegelrad 59 um die Längsachse x dreht. Wie in Figur 14 gezeigt, kann das Betätigungselement 36 am distalen Ende radial eine Schrägverzahnung 38 aufweisen, die in entsprechende Schrägverzahnungen 37 an den Maulteilen 50 greifen, um bei einer Drehung des inneren Kegelzahnrads 59 bzw. Betätigungselements 36 um die x-Achse eine Öffnungs- bzw. Schließbewegung durch Verschwenken der Maulteile 50 zu bewirken. Alternativ oder zusätzlich kann eine ähnliche Schrägverzahnung zwischen dem inneren Kegelrad 59 und dem Betätigungselement 36 vorgesehen sein, um das Betätigungselement 36 mit einer Drehung des inneren Kegelrad 59 axial in x-Richtung zu verschieben. Das Betätigungselement 36 kann 59 bzw. Betätigungselements 36 um die x-Achse eine Öffnungs- bzw. Schließbewegung durch Verschwenken der Maulteile 50 zu bewirken. Alternativ oder zusätzlich kann eine ähnliche Schrägverzahnung zwischen dem inneren Kegelrad 59 und dem Betätigungselement 36 vorgesehen sein, um das Betätigungselement 36 mit einer Drehung des inneren Kegelrad 59 axial in x-Richtung zu verschieben. Das Betätigungselement 36 kann dabei die Zangenmaulteile 50 durch axiale Bewegung in x-Richtung aufdrücken und bei axialer Bewegung in negative x-Richtung zuziehen.

Die Einzelteile des hierin beschriebenen endoskopischen Instruments 10 können im sogenannten LIGA-Verfahren hergestellt werden, d.h. mit den Verfahrensschritten Lithographie, Galvanik und Abformung. Bei den hierin beschriebenen Ausführungsbeispielen wird zwar proximalseitig bezüglich des Schafts 12 eine mechanische Schwingung erzeugt und mittels länglicher Übertragungselemente innerhalb des Schafts 12 mechanisch übertragen, allerdings ist es auch möglich, am distalen Ende des Schafts 12 ein sogenanntes mikroelektromechanisches System (MEMS) vorzusehen und elektrisch anzusteuern, um distalseitig am Schaft 12 die mechanische Schwingung zu erzeugen. In diesem Fall wäre das MEMS der Impulsgeber 55, und ein längliches Übertragungselement würde nicht benötigt.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden.

Als optional, vorteilhaft, bevorzugt, erwünscht oder ähnlich bezeichnete "kann"-Merkmale sind als optional zu verstehen.

Die beschriebenen Ausführungsformen sind als illustrative Beispiele zu verstehen und stellen keine abschließende Liste von möglichen Ausführungsformen dar.

Im Übrigen soll hierin weder der Begriff "aufweisen" zusätzliche andere Merkmale oder Verfahrensschritte ausschließen noch soll "ein" oder "eine" eine Mehrzahl ausschließen.

**Patentansprüche**

1. Endoskopisches Instrument (10) mit einem länglichen Schaft (12) und einem Instrumentenkopf (14) am distalen Schaftende, der bewegbar ist oder zumindest ein bewegbares Kopfteil (50) aufweist, mit mindestens zwei Paaren oszillatorisch beweglicher Impulsgeber (55) und mindestens zwei Übertragungsrädern (70, 202) eingerichtet zur Bewegungssteuerung des Instrumentenkopfes (14) oder des bewegbaren Kopfteils (50), wobei die Übertragungsräder (70, 202) koaxial zueinander und unabhängig voneinander drehbar auf einer quer zur Längsachse (x) des Schafts (12) verlaufenden Welle (16) angeordnet sind, mit einem ersten der Übertragungsräder (70, 202), das eingerichtet ist zur Umsetzung einer oszillatorischen Bewegung eines Impulsgebers (55) eines ersten der Impulsgeberpaare in eine gleichgerichtete Rotationsbewegung des Instrumentenkopfes (14) oder des bewegbaren Kopfteils (50) in eine erste Bewegungsrichtung und eingerichtet ist zur Umsetzung einer oszillatorischen Bewegung des anderen Impulsgebers (55) des ersten Impulsgeberpaars in eine gleichgerichtete Rotationsbewegung des Instrumentenkopfes (14) oder des bewegbaren Kopfteils (50) in eine der ersten Bewegungsrichtung entgegengesetzte zweite Bewegungsrichtung, **gekennzeichnet dadurch, dass** ein zweites der Übertragungsräder (70, 202) eingerichtet ist zur Umsetzung einer oszillatorischen Bewegung eines Impulsgebers (55) eines zweiten der Impulsgeberpaare in eine gleichgerichtete Rotationsbewegung des Instrumentenkopfes (14) oder des bewegbaren Kopfteils (50) in eine zur ersten und zweiten Bewegungsrichtung komplementäre dritte Bewegungsrichtung und eingerichtet ist zur Umsetzung einer oszillatorischen Bewegung des anderen Impulsgebers (55) des zweiten Impulsgeberpaars in eine gleichgerichtete Rotationsbewegung des Instrumentenkopfes (14) oder des bewegbaren Kopfteils (50) in eine der dritten Bewegungsrichtung entgegengesetzte vierte Bewegungsrichtung, und dadurch, dass mindestens das erste Übertragungsrad (70, 202) mit einem Kegelrad (59, 60) zur Bewegungssteuerung des Instrumentenkopfes (14) oder des bewegbaren Kopfteils (50) gekoppelt ist, wobei das Kegelrad (59, 60) um die Längsachse (x) des Schafts (12) drehbar ist.

2. Instrument (10) nach Anspruch 1, mit einem ersten Kopplungselement (74, 208), wobei das erste Kopplungselement (74, 208) während einer ersten Phase der oszillatorischen Bewegung des einen Impulsgebers (55) des ersten Impulsgeberpaars in eine erste Impulsrichtung mit dem ersten Übertragungsrad (70, 202) kraftschlüssig gekoppelt ist und während einer zweiten Phase oszillatorischen Bewegung des einen Impulsgebers (55) des ersten Impulsgeberpaars in eine der ersten Impulsrichtung entgegengesetzte zweite Impulsrichtung nicht mit dem ersten Übertragungsrad (70, 202) kraftschlüssig gekoppelt ist, sodass sich das erste Übertragungsrad (70, 202) bei oszillatorischer Bewegung des einen Impulsgebers (55) des ersten Impulsgeberpaars in eine gleichgerichtete erste Drehrichtung bewegt, welche die erste Bewegungsrichtung am distalen Schaftende zur Bewegungssteuerung des Instrumentenkopfes (14) oder des bewegbaren Kopfteils (50) definiert.

3. Instrument (10) nach Anspruch 1 oder 2, wobei eine Phasenverschiebung zwischen der oszillatorischen Bewegung des einen Impulsgebers (55) des ersten Impulsgeberpaars und des anderen Impulsgebers (55) des ersten Impulsgeberpaars die erste und die zweite Bewegungsrichtung zur Bewegungssteuerung des Instrumentenkopfes (14) oder des bewegbaren Kopfteils (50) definiert.

4. Instrument (10) nach einem der vorhergehenden Ansprüche, mit einem zweiten Kopplungselement (75, 208), wobei das zweite Kopplungselement (75, 208) während einer ersten Phase der oszillatorischen Bewegung des anderen Impulsgebers (55) des ersten Impulsgeberpaars in eine erste Impulsrichtung mit dem ersten Übertragungsrad (70, 202) kraftschlüssig gekoppelt ist und während einer zweiten Phase der oszillatorischen Bewegung des zweiten Impulsgebers (55) in eine der ersten Impulsrichtung entgegengesetzten zweiten Impulsrichtung nicht mit dem ersten Übertragungsrad (70, 202) kraftschlüssig gekoppelt ist, sodass sich das erste Übertragungsrad (70, 202) bei oszillatorischer Bewegung des anderen Impulsgebers (55) des ersten Impulsgeberpaars in eine gleichgerichtete zweite Drehrichtung bewegt, welche die zweite Bewegungsrichtung zur Bewegungssteuerung des Instrumentenkopfes (14) und/oder des bewegbaren Kopfteils (50) definiert.

5. Instrument (10) nach Anspruch 4, wobei das erste und das zweite Kopplungselement (74, 75, 208) an jeweils diametral gegenüberliegenden Seiten des ersten Übertragungsrads (70, 202) mit diesem kraftschlüssig koppelbar sind.

6. Instrument (10) nach einem der vorhergehenden Ansprüche, mit drei Paaren oszillatorisch beweglicher Impulsgeber (55) und drei Übertragungsrädern (70, 202), wobei die Übertragungsräder (70, 202) koaxial zueinander und unabhängig voneinander drehbar auf einer quer zur Längsachse (x) des Schafts (12) verlaufenden Welle (16) angeordnet sind, wobei ein drittes der Übertragungsräder (70, 202) zur Umsetzung einer oszillatorischen Bewegung eines Impulsgebers (55) eines dritten der Impulsgeberpaare in eine gleichgerichtete Rotationsbewegung in eine zur ersten, zweiten, dritten und vierten Bewegungsrichtung komplementäre fünfte Bewegungsrichtung zur Bewegungssteuerung des Instrumentenkopfes (14) oder des bewgbaren Kopfteils (50) eingerichtet ist und zur Übertragung einer oszillatorischen Bewegung des anderen Impulsgebers (55) des dritten Impulsgeberpaars in eine gleichgerichtete Rotationsbewegung in eine der fünften Bewegungsrichtung entgegengesetzte sechste Bewegungsrichtung zur Bewegungssteuerung des Instrumentenkopfes (14) oder des bewgbaren Kopfteils (50) eingerichtet ist.

7. Instrument (10) nach einem der vorhergehenden Ansprüche, mit mindestens einem piezoelektrischen Aktor zum oszillatorischen Antrieb mindestens eines der Impulsgeber (55).

8. Instrument (10) nach einem der vorhergehenden Ansprüche, wobei die gleichgerichtete Rotationsbewegung in die erste und zweite Bewegungsrichtung, die dritte und vierte Bewegungsrichtung oder die fünfte und sechste Bewegungsrichtung eine Nickbewegung des Instrumentenkopfes (14) oder eine Arbeitsbewegung eines bewegbaren Kopfteils (55) bewirkt.

9. Instrument (10) nach einem der vorhergehenden Ansprüche, wobei die gleichgerichtete Rotationsbewegung in die erste und zweite Bewegungsrichtung, die dritte und vierte Bewegungsrichtung oder die fünfte und sechste Bewegungsrichtung eine Rollbewegung des Instrumentenkopfes (14) um eine Längsachse (x) des Schafts (12) oder eine kontinuierliche Arbeitsdrehbewegung eines bewegbaren Kopfteils (55) bewirkt.

10. Instrument (10) nach einem der vorhergehenden Ansprüche, wobei die gleichgerichtete Rotationsbewegung in die erste, dritte oder fünfte Bewegungsrichtung eine Öffnungsbewegung und in die zweite, vierte oder sechste Bewegungsrichtung eine Schließbewegung eines Zangen- oder Scherenmaulteils (50) des Instrumentenkopfs (14) bewirkt.

**Claims**

1. An endoscopic instrument (10) with an elongate shank (12) and with an instrument head (14) at the distal shank end, said head being movable or comprising at least one movable head part (50), with at least two pairs of oscillatorily movable impulse generators (55) and at least two transmission wheels (70, 202) configured for the movement control of the instrument head (14) or of the movable head part (50), wherein the transmission wheels (70, 202) are arranged coaxially to one another and rotatably independently of one another on a shaft (16) which runs transversely to the longitudinal axis (x) of the shank (12),

with a first of the transmission wheels (70, 202) which is configured for converting an oscillatory movement of an impulse generator (55) of a first of the impulse generator pairs into an equally directed rotation movement of the instrument head (14) or of the movable head part (50) in a first movement direction and is configured for converting an oscillatory movement of the other impulse generator (55) of the first impulse generator pair into an equally directed rotation movement of the instrument head (14) or of the movable head part (50) in a second movement direction which is opposite to the first movement direction,

**characterised in that** a second of the transmission wheels (70, 202) is configured for converting an oscillatory movement of an impulse generator (55) of a second of the impulse generator pairs into an equally directed rotation movement of the instrument head (14) or of the movable head part (50) in a third movement direction which is complementary to the first and second movement direction and is configured for converting an oscillatory movement of the other impulse generator (55) of the second impulse generator pair into an equally directed rotation movement of the instrument head (14) or of the movable head part (50) in a fourth movement direction which is opposite to the third movement direction,

and **in that**

at least the first transmission wheel (70, 202) is coupled to a bevel wheel (59, 60) for the movement control of the instrument head (14) or of the movable head part (50), wherein the bevel wheel (59, 60) is rotatable about the longitudinal axis (x) of the shank (12).

2. An instrument (10) according to claim 1, with a first coupling element (74, 208), wherein the first coupling element (74, 208) during a first phase of the oscillatory movement of the one impulse generator (55) of the first impulse generator pair in a first impulse direction is non-positively coupled to the first transmission wheel (70, 202) and during a second phase of the oscillatory movement of the one impulse generator (55) of the first impulse generator pair in a second impulse direction which is opposite to the first impulse direction is not non-positively coupled to the first transmission wheel (70, 202), so that the first transmission wheel (70, 202) moves in an equally directed first rotation direction given an oscillatory movement of the one impulse generator (55) of the first impulse generator pair, said equally directed first rotation direction defining the first movement direction at the distal shank end for the movement control of the instrument head (14) or of the movable head part (50).

3. An instrument (10) according to claim 1 or 2, wherein a phase shift between the oscillatory movement of the one impulse generator (55) of the first impulse generator pair and of the other impulse generator (55) of the first impulse generator pair defines the first and the second movement direction for the movement control of the instrument head (14) or of the movable head part (50).

4. An instrument (10) according to one of the preceding claims, with a second coupling element (75, 208), wherein the second coupling element (75, 208) during a first phase of the oscillatory movement of the other impulse generator (55) of the first impulse generator pair in a first impulse direction is non-positively coupled to the first transmission wheel (70, 202) and during a second phase of the oscillatory movement of the second impulse generator (55) in a second impulse direction which is opposite the first impulse direction is not non-positively coupled to the first transmission wheel (70, 202), so that given an oscillatory movement of the other impulse generator (55) of the first impulse generator pair, the first transmission wheel (70, 202) moves in an equally directed second rotation direction which defines the second movement direction for the movement control of the instrument head (14) and/or of the movable head part (50).

5. An instrument (10) according to claim 4, wherein the first and the second coupling element (74, 75, 208) can be non-positively coupled to the first transmission wheel (70, 202) at sides of this which are diametrically opposite.

6. An instrument (10) according to one of the preceding claims, with three pairs of oscillatorily movable impulse generators (55) and three transmission wheels (70, 202), wherein the transmission wheels (70, 202) are arranged coaxially to one another and rotatably independently of one another on a shaft (16) which runs transversely to the longitudinal axis (x) of the shank (12), wherein a third of the transmission wheels (70, 202) is configured for converting an oscillatory movement of an impulse generator (55) of a third of the impulse generator pairs into an equally directed rotation movement in a fifth movement direction which is complementary to the first, second, third and fourth movement direction, for the movement control of the instrument head (14) or of the movable head part (50) and is configured for converting an oscillatory movement of the other impulse generator (55) of the third impulse generator pair into an equally directed rotation movement in a sixth movement direction which is opposite to the fifth movement direction, for the movement control of the instrument head (14) or of the movable head part (50).

7. An instrument (10) according to one of the preceding claims, with at least one piezoelectric actuator for the oscillatory drive of at least one of the impulse generators (55).

8. An instrument (10) according to one of the preceding claims, wherein the equally directed rotation movement in the first and second movement direction, the third and fourth movement direction or the fifth and sixth movement direction effects a nod-movement of the instrument head (14) or a working movement of a movable head part (55).

9. An instrument (10) according to one of the preceding claims, wherein the equally directed rotation movement in the first and second movement direction, the third and fourth movement direction or the fifth and sixth movement direction effects a rolling movement of the instrument head (14) about a longitudinal axis (x) of the shank (12) or a continuous working rotation movement of a movable head part (55).

10. An instrument (10) according to one of the preceding claims, wherein the equally directed rotation movement in the first, third or fifth movement direction effects an opening movement and the second, fourth or sixth movement direction effects a closure movement of a forceps or scissors jaw part (50) of the instrument head (14).

**Revendications**

1. Instrument endoscopique (10) comprenant
une tige allongée (12) et une tête d'instrument (14) à l'extrémité distale de la tige, qui est mobile ou comporte au moins une partie de tête (50) mobile,
au moins deux paires de générateurs d'impulsions (55) à mouvement oscillatoire et au moins deux roues de transmission (70, 202) agencées pour la commande de mouvement de la tête d'instrument (14) ou de la partie de tête (50) mobile, les roues de transmission (70, 202) étant montées sur un arbre (16) s'étendant transversalement à l'axe longitudinal (x) de la tige (12) de manière mutuellement coaxiale et en pouvant pivoter indépendamment l'une de l'autre,
une première des roues de transmission (70, 202) étant agencée pour la conversion d'un mouvement oscillatoire d'un générateur d'impulsions (55) d'une première des paires de générateurs d'impulsions en un mouvement de rotation unidirectionnel de la tête d'instrument (14) ou de la partie de tête (50) mobile dans une première direction de mouvement, et étant agencée pour la conversion d'un mouvement oscillatoire de l'autre générateur d'impulsions (55) de la première paire de générateurs d'impulsions en un mouvement de rotation unidirectionnel de la tête d'instrument (14) ou de la partie de tête (50) mobile dans une seconde direction de mouvement opposée à la première direction de mouvement, **caractérisé en ce qu'**une seconde des roues de transmission (70, 202) est agencée pour convertir un mouvement oscillatoire d'un générateur d'impulsions (55) d'une seconde des paires de générateurs d'impulsions en un mouvement de rotation unidirectionnel de la tête d'instrument (14) ou de la partie de tête (50) mobile dans une troisième direction de mouvement complémentaire de la première et de la seconde directions de mouvement, et agencée pour convertir un mouvement oscillatoire de l'autre générateur d'impulsions (55) de la seconde paire de générateurs d'impulsions en un mouvement de rotation unidirectionnel de la tête d'instrument (14) ou de la partie de tête (50) mobile dans une quatrième direction de mouvement opposée à la troisième direction de mouvement, et **en ce qu'**au moins la première roue de transmission (70, 202) est couplée à une roue conique (59, 60) pour la commande de mouvement de la tête d'instrument (14) ou de la partie de tête (50) mobile, la roue conique (59, 60) pouvant tourner autour de l'axe longitudinal (x) de la tige (12).

2. Instrument (10) selon la revendication 1, comprenant un premier élément de couplage (74, 208), le premier élément de couplage (74, 208) étant, lors d'une première phase du mouvement oscillatoire d'un générateur d'impulsions (55) de la première paire de générateurs d'impulsions dans une première direction d'impulsion, couplé par adhérence à la première roue de transmission (70, 202), et n'étant pas, lors d'une seconde phase de mouvement oscillatoire dudit générateur d'impulsions (55) de la première paire de générateurs d'impulsions dans une seconde direction d'impulsion opposée à la première direction d'impulsion, couplé par adhérence à la première roue de transmission (70, 202), de sorte que la première roue de transmission (70, 202) se déplace, lors du mouvement oscillatoire d'un générateur d'impulsions (55) de la première paire de générateurs d'impulsions, selon une première direction de rotation unidirectionnelle qui définit la première direction de mouvement à l'extrémité distale de la tige pour la commande de mouvement de la tête d'instrument (14) ou de la partie de tête (50) mobile.

3. Instrument (10) selon la revendication 1 ou 2, dans lequel un décalage de phase entre le mouvement oscillatoire d'un générateur d'impulsions (55) de la première paire de générateurs d'impulsions et de l'autre générateur d'impulsions (55) de la première paire de générateurs d'impulsions définit la première et la seconde directions de mouvement pour la commande de mouvement de la tête d'instrument (14) ou de la partie de tête (50) mobile.

4. Instrument (10) selon l'une des revendications précédentes, comprenant un second élément de couplage (75, 208), le second élément de couplage (75, 208) étant, lors d'une première phase du mouvement oscillatoire de l'autre générateur d'impulsions (55) de la première paire de générateurs d'impulsions dans une première direction d'impulsion, couplé par adhérence à la première roue de transmission (70, 202) et n'étant pas, lors d'une seconde phase du mouvement oscillatoire du second générateur d'impulsions (55) dans une seconde direction d'impulsion opposée à la première direction d'impulsion, couplé par adhérence à la première roue de transmission (70, 202), de sorte que la première roue de transmission (70, 202) se déplace, lors du mouvement oscillatoire de l'autre générateur d'impulsions (55) de la première paire de générateurs d'impulsions, dans une seconde direction de rotation unidirectionnelle qui définit la seconde direction de mouvement pour la commande de mouvement de la tête d'instrument (4) ou de la partie de tête (50) mobile.

5. Instrument (10) selon la revendication 4, dans lequel le premier et le second éléments de couplage (74, 75, 208) peuvent, au niveau de faces diamétralement opposées de la première roue de transmission (70, 202), être couplés par adhérence à cette dernière.

6. Instrument (10) selon l'une des revendications précédentes, comprenant trois paires de générateurs d'impulsions (55) à mouvement oscillatoire et trois roues de transmission (70, 202), les roues de transmission (70, 202) étant agencées sur un arbre (16) s'étendant transversalement à l'axe longitudinal (x) de la tige (12) de manière mutuellement coaxiale et en pouvant pivoter indépendamment l'une de l'autre, une troisième des roues de transmission (70, 202) étant agencée pour convertir un mouvement oscillatoire d'un générateur d'impulsions (55) d'une troisième des paires de générateurs d'impulsions en un mouvement de rotation unidirectionnel dans une cinquième direction de mouvement complémentaire de la première, de la seconde, de la troisième et de la quatrième directions de mouvement pour la commande de mouvement de la tête d'instrument (14) et de la partie de tête (50) mobile, et agencée pour la transmission d'un mouvement oscillatoire de l'autre générateur d'impulsions (55) de la troisième paire de capteurs d'impulsion en un mouvement de rotation unidirectionnel dans une sixième direction de mouvement opposée à la cinquième direction de mouvement pour la commande de mouvement de la tête d'instrument (14) ou de la partie de tête (50) mobile.

7. Instrument (10) selon l'une des revendications précédentes, comprenant au moins un actionneur piézoélectrique pour l'entraînement oscillatoire d'au moins l'un des générateurs d'impulsions.

8. Instrument (10) selon l'une des revendications précédentes, dans lequel le mouvement de rotation unidirectionnel dans la première et seconde directions de mouvement, la troisième et quatrième directions de mouvement ou la cinquième et sixième directions de mouvement provoque un mouvement de tangage de la tête d'instrument (14) ou un mouvement de travail d'une partie de tête (55) mobile.

9. Instrument (10) selon l'une des revendications précédentes, dans lequel le mouvement de rotation unidirectionnel dans la première et seconde directions de mouvement, la troisième et quatrième directions de mouvement, ou la cinquième et sixième directions de mouvement provoque un mouvement de roulis de la tête d'instrument (14) autour d'un axe longitudinal (x) de la tige (12) ou un mouvement de rotation de travail continu d'une partie de tête (55) mobile.

10. Instrument (10) selon l'une des revendications précédentes, dans lequel le mouvement de rotation unidirectionnel dans la première, la troisième ou la cinquième direction de mouvement provoque un mouvement d'ouverture et provoque, dans la seconde, quatrième ou sixième direction de mouvement, un mouvement de fermeture d'une partie (50) de la tête d'instrument (14) formant mâchoire de pince ou de ciseaux.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7297142 B2
- US 2014309625 A1
- US 5372124 A
- JP 5253852 B